# FASCICULE DE BREVET EUROPEEN

(11) **EP 2 136 848 B1**
(45) Date de publication et mention de la délivrance du brevet: **21.11.2012**
(21) Numéro de dépôt: 08787968.0
(22) Date de dépôt: 16.04.2008
(51) Int. Cl.: A61L 2/26, A61B 19/00, A61L 2/00

(54) **ENCEINTE ÉTANCHE DE DÉCONTAMINATION D'UN APPAREIL MÉDICAL**
VERSIEGELTES GEHÄUSE ZUR DEKONTAMINATION MEDIZINISCHER GERÄTE
SEALED ENCLOSURE FOR THE DECONTAMINATION OF A MEDICAL APPARATUS

(30) Priorité: 18.04.2007 FR 0754546
(43) Date de publication de la demande: 30.12.2009
(73) Titulaire: Germitec, 75008 Paris (FR)
(72) Inventeur: DESHAYS, Clément, F-07120 Ruoms (FR)
(74) Mandataire: Jacobson, Claude
(86) Numéro de dépôt international: PCT/FR2008/000540
(87) Numéro de publication internationale: WO 2008/142299

(56) Documents cités:
- EP-A- 0 839 537
- WO-A-2005/048041
- FR-A- 2 753 905
- FR-A- 2 890 566
- FR-A- 2 890 865
- US-A- 4 772 795
- US-A- 5 641 464

## Description

L'invention concerne une enceinte du type comportant une base, au moins une paroi latérale et un capot supérieur délimitant un volume de décontamination, une desdites au moins une paroi latérale comportant une ouverture d'accès au volume de décontamination s'étendant jusqu'au capot supérieur, le capot supérieur comportant un passage de câble ouvert vers ladite ouverture et un organe de suspension de câble au travers dudit passage de câble.

Les enceintes de ce type sont connues et utilisées par exemple pour la désinfection de la partie active d'une sonde médicale destinée à fonctionner avec un appareil d'imagerie. Une telle enceinte est connue par exemple par le document EP-A-839537.

La désinfection est par exemple effectuée par émission d'un rayonnement UV, par pulvérisation d'un liquide de désinfection sous forme de microgouttelettes ou par injection d'un gaz de désinfection dans le volume de décontamination de l'enceinte.

Dans une telle enceinte, la partie active de la sonde doit être suspendue dans le volume de décontamination afin de bénéficier de la plus grande surface de désinfection possible. En outre, il est préférable que le câble reliant la partie active de la sonde à l'appareil avec lequel la sonde est utilisée ne soit pas disposé intégralement dans l'enceinte afin de protéger la partie de connectique à l'appareil d'imagerie de ce câble et pour éventuellement permettre de ne pas débrancher la sonde de l'appareil d'imagerie lorsqu'elle est désinfectée.

Une telle réalisation nécessite donc de prévoir un passage de câble permettant d'introduire la partie active de la sonde dans l'enceinte et laisser une partie du câble en dehors de cette enceinte. Il se pose alors un problème d'étanchéité de l'enceinte de décontamination, le principe actif pouvant s'échapper de l'enceinte par le passage de câble.

Un des buts de l'invention est de proposer une enceinte de décontamination permettant d'assurer une étanchéité sûre de l'enceinte lorsque la sonde est disposée à l'intérieur de celle-ci.

A cet effet, l'invention concerne une enceinte de décontamination du type précité, caractérisée en ce qu'elle comprend un volet d'obturation mobile entre une position de fermeture du passage de câble, dans laquelle le volet ferme le passage de câble de manière hermétique, et une position d'ouverture du passage de câble.

Le volet de fermeture du passage de câble permet de garantir une étanchéité parfaite de l'enceinte lorsqu'elle est fermée, bien que le câble de la sonde sorte de celle-ci.

Selon d'autres caractéristiques de l'enceinte de décontamination de l'invention :
- le passage de câble comporte une fente aménagée dans le capot supérieur ;
- le volet est porté par le capot ;
- l'enceinte comprend une porte de fermeture de l'ouverture d'accès au volume de décontamination, ladite porte étant agencée pour que sa fermeture entraîne le volet mobile vers sa position de fermeture du passage de câble ;
- le volet est solidaire de la partie supérieure de la porte et est agencé pour fermer le passage du câble de manière hermétique lorsque la porte est fermée ;
- l'organe de suspension de câble comporte une pince ;
- l'organe de suspension de câble comporte un crochet ;
- l'organe de suspension de câble comporte un tube ouvert apte à s'emboîter de manière hermétique sur une portion de câble de l'instrument médical dont le diamètre est dans une plage de diamètres prédéterminés ;
- l'organe de suspension comporte une pièce de réception d'une pièce de suspension prévue sur le câble de l'instrument médical, ladite pièce de réception et ladite pièce de suspension formant un ensemble d'assemblage constitué d'une pièce mâle et d'une pièce femelle ;
- l'enceinte comprend des moyens de lecture d'une étiquette d'identification électronique prévue sur un câble de l'instrument médical, ladite étiquette étant disposée à proximité de la partie active à désinfecter de l'instrument médical, lesdits moyens de lecture étant agencés pour être à proximité de l'étiquette d'identification lorsque le câble est suspendu à l'organe de suspension de câble ; et
- les moyens de lecture d'une étiquette d'identification électronique comprennent un lecteur RFID de faible portée.

D'autres aspects et avantages de l'invention apparaîtront au cours de la description qui suit, faite à titre d'exemple et en référence aux figures annexées.

La Fig. 1 est une représentation schématique en perspective d'un appareil d'imagerie médical auquel est fixée une enceinte de décontamination selon l'invention, une sonde étant disposée dans l'enceinte.

La Fig. 2 est une représentation schématique en perspective de l'enceinte de la Fig. 1 dans laquelle une sonde est disposée.

La Fig. 3 est une représentation schématique en perspective de l'appareil d'imagerie médical de la Fig. 1, l'enceinte étant ouverte.

La Fig. 4 est une représentation schématique en perspective de l'enceinte ouverte de la Fig. 3.

La Fig. 5 est une représentation schématique en coupe de l'ensemble d'assemblage du câble de la sonde et de l'enceinte, en cours d'assemblage.

La Fig. 6 est une représentation schématique en coupe selon la ligne VI-VI de la Fig. 2.

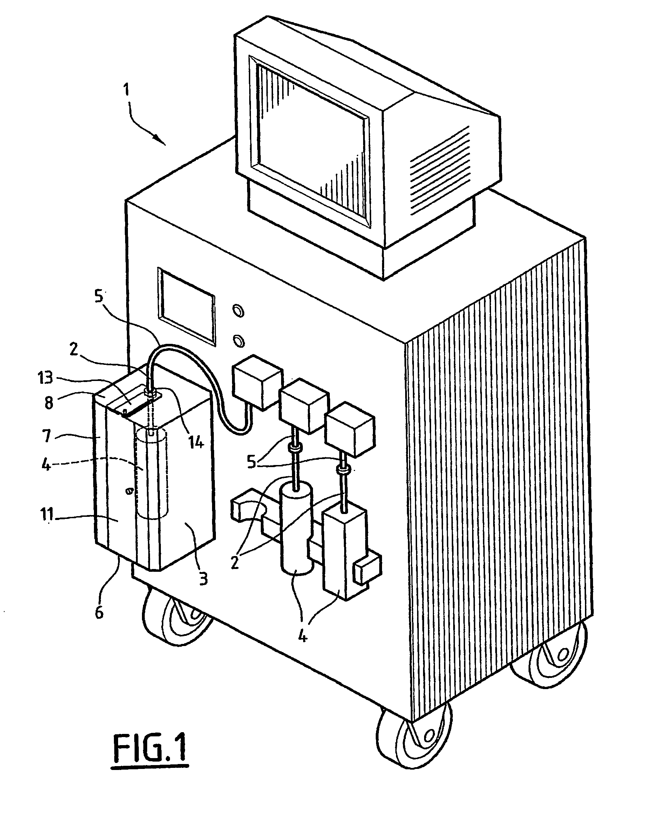

En référence à la Fig. 1, on décrit un appareil d'imagerie médical 1 susceptible de fonctionner avec une pluralité de sondes 2 et auquel est fixée une enceinte de décontamination 3 de la partie active 4 des sondes 2.

Dans le mode de réalisation représenté sur les figures, l'enceinte de décontamination 3 est fixée à l'appareil d'imagerie 1. Selon d'autres modes de réalisation, l'enceinte 3 est indépendante de l'appareil d'imagerie 1 ou est fixée à un autre appareil médical.

Dans le mode de réalisation représenté sur les figures, trois sondes 2 sont connectées à l'appareil d'imagerie 1. Selon d'autres modes de réalisation, un nombre différent de sondes peut être prévu. L'appareil d'imagerie 1 est connu en lui-même et ne sera pas décrit plus en détail ici. Une sonde 2 comprend une partie active 4 qui doit être désinfectée avant chaque utilisation et un câble 5 de connexion à l'appareil médical 1 et dont la partie de connectique ne doit pas être exposée au principe actif utilisé au cours de la désinfection. La sonde 2 est connue en elle-même et ne sera pas décrite plus en détail ici.

L'enceinte 3 comprend une base 6 formant le fond de l'enceinte, au moins une paroi latérale 7 formant le corps de l'enceinte et un capot supérieur 8. La paroi latérale 7 s'étend entre la base 6 et le capot supérieur de sorte à délimiter un volume de décontamination 9 dans lequel la partie active 4 de la sonde 2 est disposée afin d'être désinfectée.

Selon le mode de réalisation représenté sur les figures, l'enceinte 3 est sensiblement parallélépipédique et comprend quatre parois latérales 7. Selon d'autres modes de réalisation, l'enceinte 3 peut être sensiblement cylindrique de section circulaire ou autre.

L'enceinte 3 comprend des moyens (non représentés) de diffusion d'un principe actif de désinfection dans le volume de décontamination 9. Ces moyens sont par exemple une source d'émission d'UV, une buse de pulvérisation de microgouttelettes d'un liquide de désinfection ou une buse d'injection d'un gaz de désinfection. Les moyens de diffusion sont agencés pour que la partie active 4 de la sonde 2 soit uniformément exposée au principe actif de désinfection.

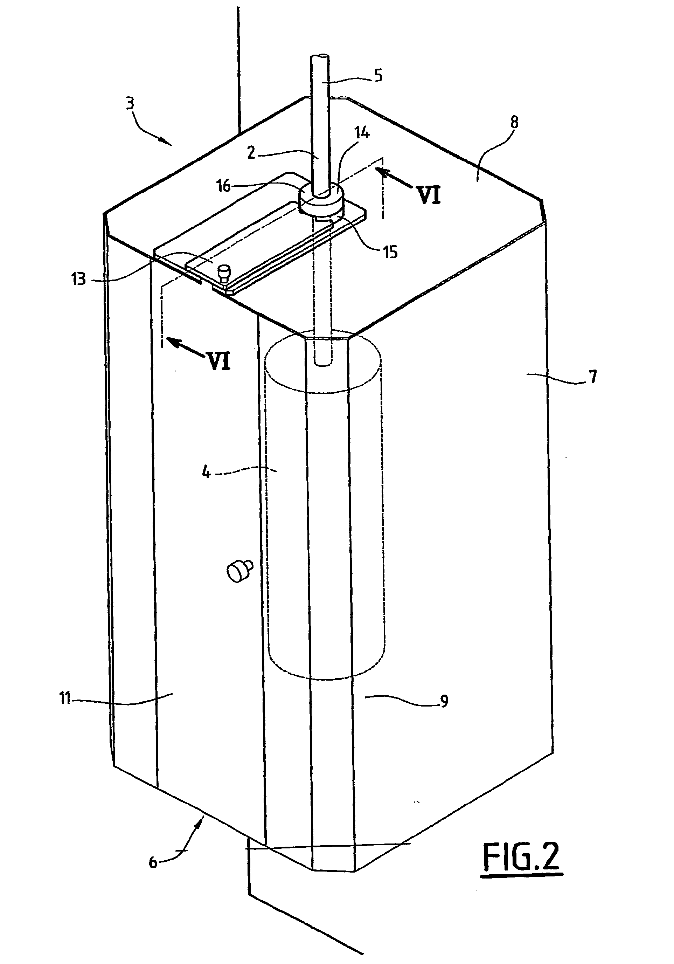

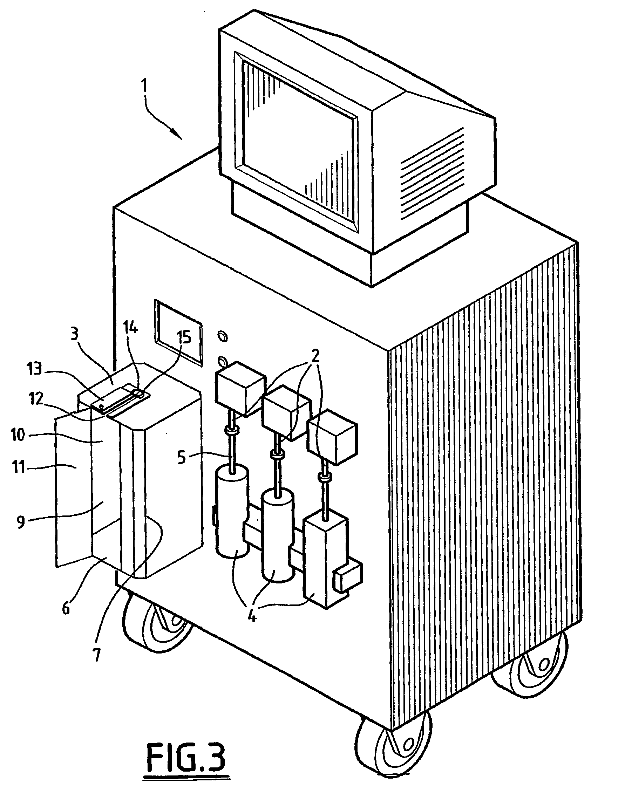

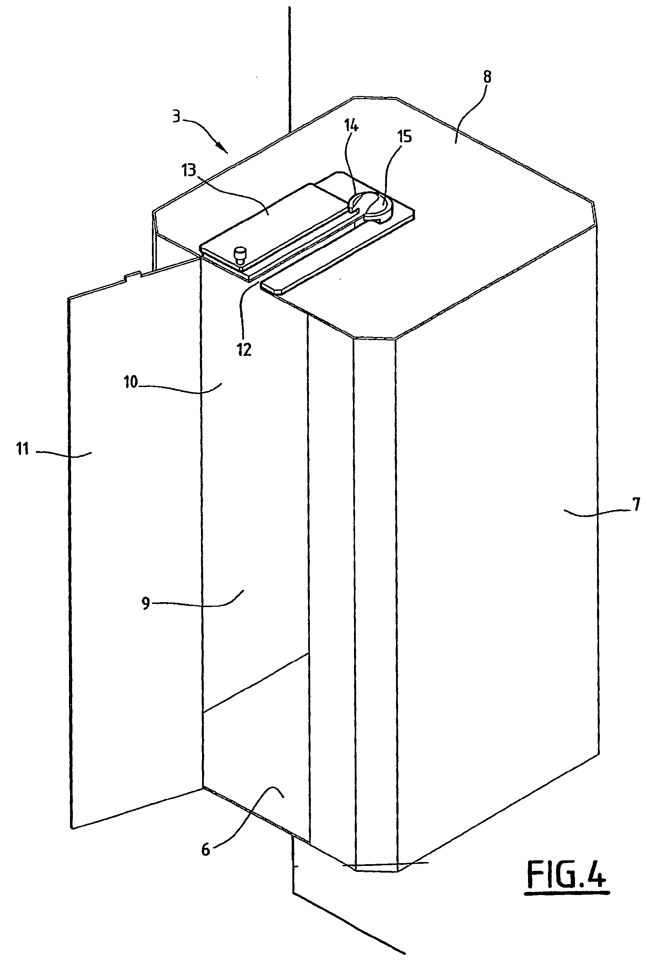

Dans le mode de réalisation représenté sur les figures, la paroi 7 opposée à l'appareil d'imagerie comprend une ouverture 10 qui peut être fermée par une porte 11. La porte est représentée en position fermée sur les Figs. 1 et 2 et en position ouverte sur les Figs. 3 et 4. L'ouverture 10 présente des dimensions adaptées pour le passage de la partie active 4 de la sonde 2. La porte 11 est ouverte pour permettre de disposer la partie active 4 d'une sonde 2 dans le volume de décontamination 9 puis est fermée lors de la désinfection. Lorsque la porte 11 est fermée, le volume 9 est fermé de façon hermétique de sorte à éviter les fuites du principe actif de désinfection vers l'extérieur de l'enceinte 3.

Le capot 8 comprend un passage de câble 12 ouvert vers l'ouverture 10 pour permettre le passage du câble 5 de la sonde 2 lorsque la partie active 4 est introduite dans le volume de décontamination 9. Le passage de câble 12 comporte une fente aménagée dans le capot 8 et s'étendant jusqu'à une partie centrale du capot 8, comme représenté sur les Figs. 3 et 4.

Selon un mode de réalisation représenté sur les figures, le capot 8 comporte en outre un volet d'obturation 13 mobile entre une position de fermeture (Figs. 1 et 2) du passage de câble 12 et une position d'ouverture (Figs. 3 et 4) du passage de câble 12. Le volet 13 ferme le passage du câble 12 de manière hermétique lorsqu'il est en position de fermeture. Ainsi, lorsque la partie active 4 de la sonde 2 a été introduite dans le volume de décontamination 9, la porte 11 et le volet 13 sont mis en position de fermeture de sorte à isoler hermétiquement le volume 9 de l'extérieur de l'enceinte et permettre une désinfection sans risque de la partie active 4. Selon un mode de réalisation, la porte 11 est agencée pour que sa fermeture entraîne le volet mobile 13 vers sa position de fermeture du passage de câble 12. Ainsi, il suffit à l'opérateur de fermer la porte 11 pour que le volet se place automatiquement en position de fermeture, ce qui évite les erreurs de manipulation si l'opérateur oublie d'actionner le volet 13 après avoir disposé la sonde 2 dans l'enceinte 3.

Selon un autre mode de réalisation non représenté, le volet 13 est solidaire de la partie supérieure de la porte 11 et est agencé pour fermer le passage du câble 12 de manière hermétique lorsque la porte est fermée. Un tel mode de réalisation est particulièrement simple à réaliser et ne nécessite pas de moyens de transmission de mouvement entre la porte 11 et le volet 13, contrairement au cas où le volet 13 est porté par le capot 8.

Le capot 8 comprend en outre un organe 14 de suspension de câble au travers du passage de câble 12. L'organe 14 de suspension de câble permet de maintenir le câble 5 de la sonde 2 de sorte que la partie active 4 soit suspendue dans une partie sensiblement centrale du volume de décontamination 9, comme représenté sur les Figs. 1, 2 et 6. On évite ainsi que la partie active 4 touche les parois 7 de l'enceinte 3 et on assure une exposition uniforme de la partie active au principe actif de désinfection.

Selon le mode de réalisation représenté sur les figures, l'organe de suspension 14 comprend une pièce de réception 15 d'une pièce de suspension 16 prévue sur le câble 5 de la sonde 2. La pièce de réception 15 et la pièce de suspension 16 forment un ensemble d'assemblage constitué d'une pièce mâle et d'une pièce femelle. Selon le mode de réalisation représenté sur les figures, la pièce mâle est la pièce de réception 15 et la pièce femelle est la pièce de suspension 16.

Selon un mode de réalisation, l'ensemble d'assemblage est agencé pour que le câble 5 soit orienté d'une façon particulière lorsque la pièce de suspension 16 est fixée sur la pièce de réception 15. Par exemple, l'orientation du câble est obtenue par vissage jusqu'à une position de butée de la pièce de suspension 16 sur la pièce de réception 15.

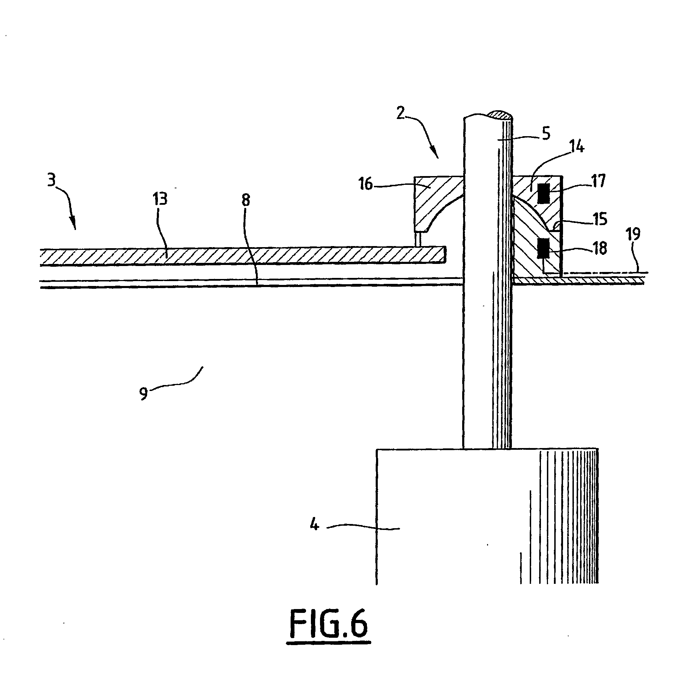

Comme représenté sur la Fig. 6, le volet 13 et l'ensemble d'assemblage sont agencés pour que l'enceinte 3 soit fermée hermétiquement lorsque la pièce de suspension 16 est fixée sur la pièce de réception 15 et que le volet 13 est fermé.

Selon d'autres modes de réalisation non représentés, l'organe de suspension 14 comporte une pince, un crochet ou un tube ouvert apte à s'emboîter de manière hermétique sur une portion de câble 5 dont le diamètre est dans une plage de diamètres prédéterminés. Selon divers modes de réalisation, on prévoit des moyens d'orientation du câble 5, par exemple par des moyens d'indexation ou autres, afin que le câble présente une orientation prédéterminée lorsque la partie active 4 est suspendue dans le volume de décontamination 9.

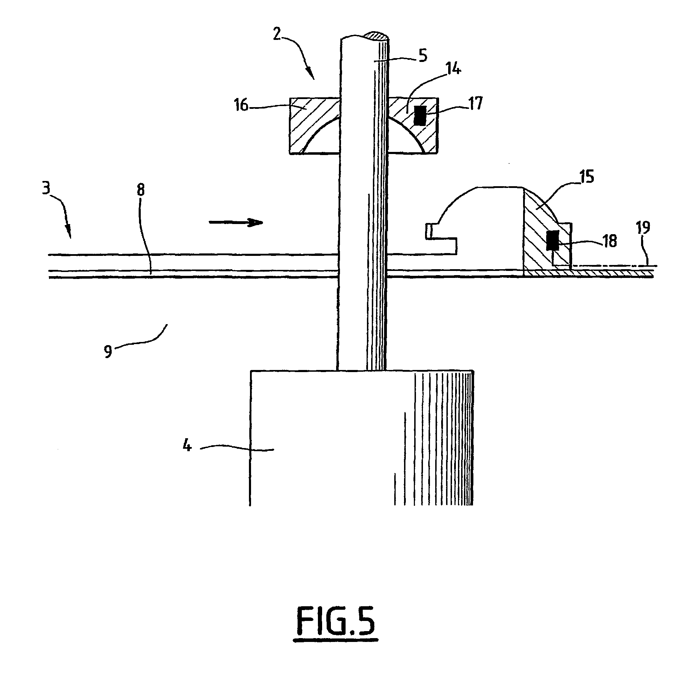

Pour assurer une traçabilité de la désinfection d'une sonde afin d'éviter tout usage illégitime d'une sonde du point de vue hygiénique, il est important de pouvoir identifier sans erreur la sonde qui est désinfectée dans l'enceinte. A cet effet, selon un mode de réalisation, la sonde 2 comprend des moyens d'identification du type étiquette d'identification électronique 17, par exemple une puce RFID. Cette puce contient des informations relatives à l'identité de la sonde. L'étiquette 17 est par exemple fixée au câble 5 à proximité de l'organe de suspension 14 lorsque la partie active 4 de la sonde 2 est suspendue dans le volume de décontamination 9. Selon un mode de réalisation, l'étiquette 17 est fixée ou noyée dans la pièce de suspension 16, comme représenté sur les Figs. 5 et 6.

L'enceinte comprend des moyens de lecture 18 de l'étiquette d'identification électronique, par exemple du type lecteur RFID. Les moyens de lecture 18 sont agencés pour être à proximité de l'étiquette d'identification 17 lorsque le câble 5 est suspendu à l'organe de suspension 15 de sorte que les moyens de lecture 18 peuvent lire les informations d'identification de l'étiquette d'identification 17. La disposition des moyens de lecture à proximité de l'étiquette d'identification peut être simplifiée par les moyens d'orientation du câble 5. Selon un mode de réalisation, les moyens de lecture 18 sont prévus au niveau de l'organe de suspension 15 ou intégrés à celui-ci, comme représenté sur les Figs. 5 et 6. Les moyens de lecture 18 sont par exemple un lecteur RFID de faible portée. Ainsi, le lecteur ne peut lire que les informations contenues dans l'étiquette d'identification fixée au câble 5 de la sonde 2 qui est suspendue dans l'enceinte 2 et non des informations de sondes voisines, telles comme les autres sondes représentées sur la Fig. 1. Les moyens de lecture 18 sont connectés à l'appareil d'imagerie 1 ou à système de traitement de données, par exemple par une connexion filaire 19. Selon une autre réalisation, les moyens de lecture sont connectés par une connexion sans fil.

Selon un autre mode de réalisation non représenté, l'étiquette d'identification électronique 17 est fixée à l'intérieur d'une saillie du câble 5, qui est introduite dans le passage de câble 12. Le passage de câble 12 comprend un logement de réception de la saillie à proximité duquel les moyens de lecture 18 sont disposés. On prévoit par exemple un logement au bout de la fente aménagée dans le capot 8 dans lequel la saillie est emboîtée lorsque le câble 5 est suspendu dans l'enceinte. Les moyens de lecture 18 à proximité du logement permettent de lire le contenu de l'étiquette d'identification 17 qui est fixée dans la saillie. L'emboîtement de la saillie dans le logement est agencé pour que l'enceinte 9 soit fermée hermétiquement lorsque la partie active 4 de la sonde est introduite dans l'enceinte.

L'étiquette d'identification 17 de la sonde 2 peut fonctionner de paire avec des moyens de mesure (non représentés) de caractéristiques de la désinfection afin d'assurer une traçabilité de la désinfection de la sonde. On peut par exemple prévoir des moyens de mesure de l'intensité du rayonnement UV et du temps d'exposition de la partie active 4 à ce rayonnement si la désinfection est réalisée par UV. Ces informations peuvent être enregistrées dans l'étiquette d'identification de la sonde ou dans l'appareil d'imagerie 1 avec l'identité de la sonde 2. On obtient ainsi un couplage des informations d'identification de la sonde et des informations de désinfection de cette sonde afin d'assurer une traçabilité de la désinfection.

Selon un mode de réalisation, l'appareil d'imagerie 1 est agencé pour ne pas fonctionner avec une sonde dont la désinfection n'a pas eu lieu ou a été insuffisante.

## Revendications

1. Enceinte de décontamination pour instruments médicaux, comportant une base (6), au moins une paroi latérale (7) et un capot supérieur (8) délimitant un volume de décontamination (9), une desdites au moins une paroi latérale ( 7) comportant une ouverture d'accès (10) au volume de décontamination (9) s'étendant jusqu'au capot supérieur (8), le capot supérieur (8) comportant un passage de câble (12) ouvert vers ladite ouverture et un organe de suspension (14) de câble au travers dudit passage de câble, ladite enceinte comprenant un volet d'obturation (13) mobile entre une position de fermeture du passage de câble (12), dans laquelle le volet (13) ferme le passage de câble (12) de manière hermétique et une position d'ouverture du passage de câble (12), ladite enceinte étant **caractérisée en ce qu'**elle comprend une porte (11) de fermeture de l'ouverture (10) d'accès au volume de décontamination (9), ladite porte étant agencée pour que sa fermeture entraîne le volet mobile (13) vers sa position de fermeture du passage de câble (12)..

2. Enceinte selon la revendication 1, **caractérisée en ce que** le passage de câble (12) comporte une fente aménagée dans le capot supérieur (8).

3. Enceinte selon la revendication 1 ou 2, **caractérisée en ce que** le volet (13) est porté par le capot (B).

4. Enceinte selon la revendication 1 ou 2, **caractérisée en ce que** le volet (13) est solidaire de la partie supérieure de la porte (11) et est agencé pour fermer le passage du câble (12) de manière hermétique lorsque la porte (11) est fermée.

5. Enceinte selon l'une quelconque des revendications 1 à 4, **caractérisée en ce que** l'organe de suspension (14) de câble comporte une pince.

6. Enceinte selon l'une quelconque des revendications 1 à 4, **caractérisée en ce que** l'organe de suspension (14) de câble comporte un crochet

7. Enceinte selon l'une quelconque des revendications 1 à 4, **caractérisée en ce que** l'organe de suspension (14) de câble comporte un tube ouvert apte à s'emboîter de manière hermétique sur une portion de câble de l'instrument médical dont le diamètre est dans une plage de diamètres prédéterminés.

8. Enceinte selon l'une quelconque des revendications 1 à 4, **caractérisée en ce que** l'organe de suspension (14) comporte une pièce de réception (15) d'une pièce de suspension (16) prévue sur le câble (5) de l'instrument médical, ladite pièce de réception (15) ladite pièce de suspension (16) étant apte à former avec un ensemble d'assemblage constitué d'une pièce mâle et d'une pièce femelle.

9. Enceinte selon l'une quelconque des revendications 1 à 8, **caractérisée en ce que** l'enceinte comprend des moyens de lecture (18) d'une étiquette d'identification électronique (17) prévue sur un câble (5) de l'instrument médical, ladite étiquette étant disposée à proximité de la partie active (4) à désinfecter de l'instrument médical, lesdits moyens de lecture (18) étant agencés pour être à proximité de l'étiquette d 'identification (17) lorsque le câble (5) est suspendu à l'organe de suspension (14) de câble.

10. Enceinte selon la revendication 9, **caractérisée en ce que** les moyens de lecture (18) d'une étiquette d'identification électronique (17) comprennent un lecteur RFID.

## Claims

1. A decontamination chamber for medical instruments, comprising a base (6), at least one side wall (7) and a top cover (8) defining a decontamination space (9), one of said at least one side wall (7) comprising an opening (10) extending as far as the top cover (8) for accessing the decontamination space (9), the top cover (8) comprising a cable passage (12) open towards said opening and a member (14) for cable suspension through said cable passage, said chamber comprising a sealing flap (13) movable between a cable passage (12) closing position, in which the flap (13) closes the cable passage (12) hermetically, and a cable passage (12) opening position, said chamber being **characterised in that** it comprises a door (11) for closing the opening (10) allowing access to the decontamination space (9), said door being arranged such that closure thereof drives the movable flap (13) towards its cable passage (12) closing position.

2. A chamber according to claim 1, **characterised in that** the cable passage (12) comprises a slot formed in the top cover (8).

3. A chamber according to claim 1 or claim 2, **characterised in that** the flap (13) is carried by the cover (8).

4. A chamber according to claim 1 or claim 2, **characterised in that** the flap (13) is integral with the top part of the door (11) and is arranged to close the cable passage (12) hermetically when the door (11) is closed.

5. A chamber according to any one of claims 1 to 4, **characterised in that** the cable suspension member (14) comprises a clip.

6. A chamber according to any one of claims 1 to 4, **characterised in that** the cable suspension member (14) comprises a hook.

7. A chamber according to any one of claims 1 to 4, **characterised in that** the cable suspension member (14) comprises an open tube suited to fitting hermetically onto a cable portion of the medical instrument whose diameter is in a range of predetermined diameters.

8. A chamber according to any one of claims 1 to 4, **characterised in that** the suspension member (14) comprises a receiving element (15) for a suspension element (16) provided on the cable (5) of the medical instrument, said receiving element (15) being suited to forming with said suspension part (16) an assembly consisting of a male part and a female part.

9. A chamber according to any one of claims 1 to 8, **characterised in that** the chamber comprises means (18) for reading an electronic identification label (17) provided on a cable (5) of the medical instrument, said label being disposed close to the active part (4) to be disinfected of the medical instrument, said reading means (18) being arranged so as to be close to the identification label (17) when the cable (5) is suspended in the cable suspension member (14).

10. A chamber according to claim 9, **characterised in that** the means (18) for reading an electronic identification label (17) comprise an RFID reader.

## Patentansprüche

1. Dekontaminierungskammer für medizinische Instrumente, eine Grundplatte (6) umfassend, mindestens eine Seitenwand (7) und eine obere Abdeckung (8), die einen Dekontaminierungsraum (9) umgrenzen, wobei eine der genannten mindestens einen Seitenwände (7) eine Zugangsöffnung (10) zum Dekontaminierungsraum (9) aufweist, die sich bis zur oberen Abdeckung (8) erstreckt, wobei die obere Abdeckung (8) eine zur genannten Öffnung offene Kabeldurchführung (12) aufweist und ein Organ zur Aufhängung (14) des Kabels durch die genannte Kabeldurchführung, wobei die genannte Kammer eine Verschlussklappe (13) umfasst, die zwischen einer Verschlussstellung der Kabeldurchführung (12), in der die Klappe (13) die Kabeldurchführung (12) hermetisch verschließt, und einer Offenstellung der Kabeldurchführung (12) bewegbar ist, wobei die genannte Kammer **dadurch gekennzeichnet ist, dass** sie eine Tür (11) zum Verschließen der Zugangsöffnung (10) zum Dekontaminierungsraum (9) umfasst, wobei die genannte Tür derart eingerichtet ist, dass sie beim Schließen die bewegliche Klappe (13) in die Verschlussstellung der Kabeldurchführung (12) mitführt.

2. Kammer nach Patentanspruch 1, **dadurch gekennzeichnet, dass** die Kabeldurchführung (12) einen Schlitz aufweist, der in der oberen Abdeckung (8) ausgebildet ist.

3. Kammer nach Patentanspruch 1 oder 2, **dadurch gekennzeichnet, dass** die Klappe (13) von der Abdeckung (B) getragen wird.

4. Kammer nach Patentanspruch 1 oder 2, **dadurch gekennzeichnet, dass** die Klappe (13) mit dem oberen Teil der Tür (11) fest verbunden und dafür eingerichtet ist, die Kabeldurchführung (12) hermetisch zu verschließen, wenn die Tür (11) geschlossen ist.

5. Kammer nach irgendeinem der Patentansprüche 1 bis 4, **dadurch gekennzeichnet, dass** das Organ (14) zur Aufhängung des Kabels eine Zange umfasst.

6. Kammer nach irgendeinem der Patentansprüche 1 bis 4, **dadurch gekennzeichnet, dass** das Organ (14) zur Aufhängung des Kabels einen Haken umfasst.

7. Kammer nach irgendeinem der Patentansprüche 1 bis 4, **dadurch gekennzeichnet, dass** das Organ (14) zur Aufhängung des Kabels ein offenes Rohr umfasst, das in der Lage ist, hermetisch auf einen Teil des Kabels des medizinischen Instrumentes gesteckt zu werden, dessen Durchmesser in einem festgelegten Durchmesserbereich liegt.

8. Kammer nach irgendeinem der Patentansprüche 1 bis 4, **dadurch gekennzeichnet, dass** das Aufhängorgan (14) ein Aufnahmestück (15) eines am Kabel (5) des medizinischen Instrumentes vorgesehenen Aufhängstückes (16) umfasst, wobei das genannte Aufnahmestück (15) in der Lage ist, mit dem genannten Aufhängstück (16) eine zusammengesetzte Einheit zu bilden, die aus einem Einsteck- und einem Aufnahmeteil besteht.

9. Kammer nach irgendeinem der Patentansprüche 1 bis 8, **dadurch gekennzeichnet, dass** die Kammer Mittel zum Lesen (18) eines elektronischen Kennzeichnungsetiketts (17) umfasst, das an einem Kabel (5) des medizinischen Intrumentes vorgesehen ist, wobei das genannte Etikett nahe dem zu desinfizierenden aktiven Teil (4) des medizinischen Instrumentes angeordnet ist, wobei die genannten Lesemittel (18) derart angeordnet sind, dass sie sich nahe dem Kennzeichnungsetikett (17) befinden, wenn das Kabel (5) am Kabelaufhängorgan (14) aufgehängt ist.

10. Kammer nach Patentanspruch 9, **dadurch gekennzeichnet, dass** die Mittel (18) zum Lesen eines elektronischen Kennzeichnungsetiketts (17) einen RFID-Leser umfassen.
